# EUROPEAN PATENT SPECIFICATION

(11) **EP 1 808 537 B1**
(45) Date of publication and mention of the grant of the patent: **11.03.2009**
(21) Application number: 06381003.0
(22) Date of filing: 16.01.2006
(51) Int. Cl.: E03D 9/03

(54) **Dual-purpose machine for air freshening and hygienic cleansing**
Abgabevorrichtung für Duft- und Desinfektionsmittel
Dispositif de distribution pour déodorisant d'air et composition désinfectante

(43) Date of publication of application: 18.07.2007
(73) Proprietor: AROMANIA FRAGANCIAS NATURALES, S.L., 50800 ZUERA (ES)
(72) Inventor: Torres, Pedro, 50800, Zuera (ES)
(74) Representative: Esteban Perez-Serrano, Maria Isabel

(56) References cited:
- US-A- 5 367 716
- US-A- 5 823 390

## Description

### OBJECT OF THE INVENTION

The present invention relates to a dual-purpose machine for air freshening and hygienic cleansing, characterised in that it has an electric motor that drives a speed reducer mechanism provided with an alternating button, the motion of which depends on the sense of rotation of the motor to press two actuators that eject the content of the two bottles placed inside the device.

### BACKGROUND OF THE INVENTION

Known in the state of the art are devices containing a hygienic cleansing liquid for urinals and toilets with a controlled dispensation. Normally these are wall-mounted units, which feed one or several urinals or toilets through one or more hoses.

Also known in the state of the art are devices containing a vaporiser that supplies an aromatic vaporised liquid in a controlled manner.

However, the existence is not known of machines that can perform those aforementioned two functions using a single motor. For this purpose, a button system has been designed that allows pressing alternately the actuators that release the liquid in the bottles included in the device.

Another disadvantage of hygienic cleansers available in the market is the difficulty of supplying a uniform amount of liquid to each urinal to which they are connected, due to the liquid outlet nozzles and the subsequent storage of the liquid in a deposit with separate cavities from which it is later fed through flexible hoses to the corresponding urinal. The invention disclosed overcomes this problem by a pipette the design of which provides an identical flow of liquid at each of its outlets and direct supply to each urinal. With this invention, the inclination of the device will not affect the volume of liquid dosed to each urinal.

It is known document US5367716 which discloses a dual-purpose machine for air freshening and hygienic cleansing, wherein an electric motor for driving a speed reducer mechanism with two bottles. Taking Into account this document the present invention solved the problem of to selectively actuate both an air freshener and a hygienic cleanser.

Also known is document US5823390 discloses a similar mechanism for dispensing only one additive into a toilet/urinal bowl.

### DESCRIPTION OF THE INVENTION

The present Invention relates to a dual-purpose machine for air freshening and hygienic cleansing, characterised in that it has an electric motor for actuating a speed reducer mechanism with an alternating button the motion of which depends on the sense of rotation of the motor to press two actuators that result in the ejection of the contents of two bottles located inside the device.

One of the bottles contains an air freshener and the other contains a hygienic cleaner.

The speed reducer mechanism consists of a drive gear with a pinion on its shaft that in turn engages an intermediate gear with a pinion on its shaft that drives the alternating button.

The alternating button has a circular sector shape with a toothed upper sector that engages the drive pinion and a lower part having two end extensions acting as buttons for each bottle. The alternating button is located between the two bottles, i.e. between the sprayer of the air freshener bottle and the dosifier of the hygienic cleaner bottle.

Therefore, a single motor depending on its sense of rotation will drive the speed reducer in one sense or the other, which will turn the button clockwise or anticlockwise, pressing either the air-freshener spray or the hygienic cleanser dosifier. The frequency and sense of rotation of the motor are controlled by a programmable electronic board.

The bottle containing the hygienic cleanser has a pipette with two aligned vertical outlets to which a flexible hose is attached. Having two aligned vertical outlets allows the liquid flow in each outlet to be identical.

The flexible hoses are guided behind the machine through partitions in which each hose runs.

Each hose runs directly to each urinal. This direct feed of the urinals means that even if the machine is inclined the liquid outlet will remain uniform, not favouring the outlet of liquid through one hose more than the other.

If one wishes to feed only one urinal, one of the pipette outlets can be blocked. It is also possible to feed more than two urinals by placing inverted-Y hoses.

The dual purpose machine for air freshening and hygienic cleansing has a housing for the batteries and a housing for the motor.

It also has a led that indicates the level of liquid in the bottles.

The outer casing has one orifice for outlet of the pulverised air freshening liquid and another orifice for the indicator led.

All internal elements of the machine are attached to the casing by clips.

### DESCRIPTION OF THE DRAWINGS

The present descriptive memory is completed by a set of drawings that illustrate an example of a preferred embodiment without limiting the invention.

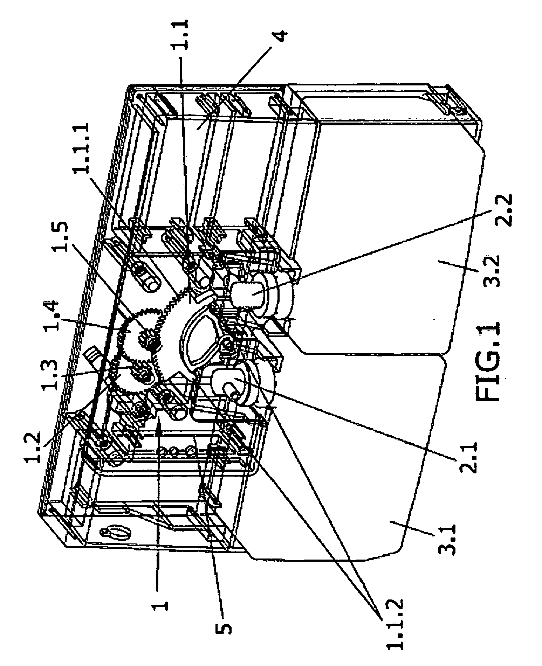

Figure 1 is a perspective view of the interior of the complete machine.

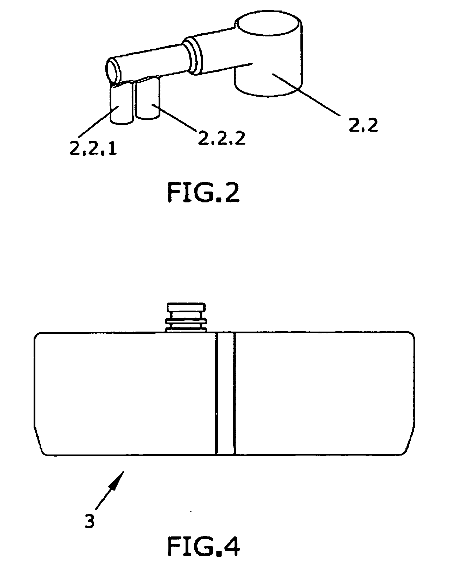

Figure 2 is a perspective representation of the pipette with the two outlets for the hygienic cleanser.
Figure 3 is a plan view of the rear part of the machine.
Figure 4 is a side view of a double bottle for air freshening not in accordance with the invention.

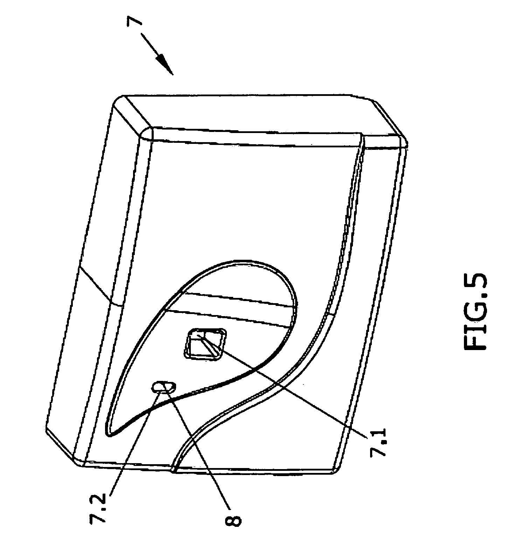

Figure 5 is a perspective view of the outer casing.

### PREFERRED EMBODIMENT OF THE INVENTION

The present invention relates to a dual-purpose machine for air freshening and hygienic cleansing, characterised in that it has an electric motor (not shown) that drives a speed reducer mechanism (1) with an alternating button (1.1) the motion of which depends on the sense of rotation of the motor to press two actuators (2.1, 2.2) that cause the ejection of the contents of the two bottles (3.1, 3.2) located inside the device.

The speed reducer mechanism (1) consists of a drive gear (1.2) with a pinion (1.3) on its shaft, which engages an intermediate gear (1.4) also with a pinion (1.5) on its shaft that drives the alternating button (1.1).

The alternating button (1.1) has a circular sector shape with an upper sector (1.1.1) that is toothed and engages the drive pinion (1.5), the ends of the lower part having two extensions (1.1.2) acting as buttons.

The bottles (3.1, 3.2) contain an air freshener (3.1) and a hygienic cleaner (3.2).

The bottle (3.2) containing the hygienic cleaner has a pipette (2.2) with two aligned vertical outlets (2.2.1, 2.2.2) to which a flexible hose is connected.

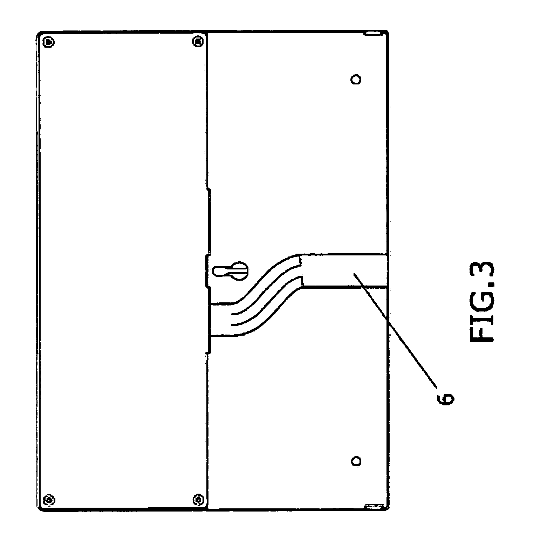

The flexible hoses are guided on the rear of the machine, see figure 3, through partitions (6) in which each hose runs.

The machine has a housing (4) for the batteries (not shown) and a housing (5) for the motor (not shown).

It also has a led (8) that indicates the level of liquid in the bottles (3.1, 3.2).

The outer casing (7) has an orifice (7.1) for outlet of the pulverised air freshening liquid and another orifice (7.2) for the indicator led (8).

All internal elements of the machine are attached to the casing by clips.

The essence of this invention is not affected by changes in the materials, shape, size and arrangement of the component elements, described in a nonlimiting manner that should allow its reproduction by an expert.

## Claims

1. Dual-purpose machine for air freshening and hygienic cleansing, which has two bottles (3.1, 3.2) and an electric motor **characterised by**
• a speed reducer mechanism (1) actuated by the electric motor;
• an alternating button (1.1), the motion of which depends on the sense of rotation of the motor; and
• two actuators (2.1, 2.2), pressed by the alternating button (1.1) which causes the ejection of the content of one of the two bottles (3.1, 3.2), respectively.

2. Dual-purpose machine for air freshening and hygienic cleansing, according to claim 1, **characterised in that** the speed reducer mechanism (1) consists of a drive gear (1.2) with a pinion (1.3) on its shaft, which engages an intermediate pinion (1.4) also with a pinion (1.5) on its shaft that drives the alternating button (1.1).

3. Dual-purpose machine for air freshening and hygienic cleansing, according to claim 2, **characterised in that** the alternating button (1.1) has a circular-sector shape with an upper sector (1.1.1) that is toothed and engages the drive pinion (1.5) of the intermediate pinion, the ends of the lower part of the button (1.1) having two extensions (1.1.2) acting as buttons.

4. Dual-purpose machine for air freshening and hygienic cleansing, according to claim 1, **characterised in that** one of the bottles (3.1) contains an air freshener and the other bottle (3.2) contains a hygienic cleaner.

5. Dual-purpose machine for air freshening and hygienic cleansing, according to claim 4, **characterised in that** the bottle (3.2) that contains the hygienic cleaner has a pipette (2.2) with two aligned vertical outlets (2.2.1, 2.2.2) to which a flexible hose is connected.

6. Dual-purpose machine for air freshening and hygienic cleansing, according to claim 5, **characterised in that** the flexible hoses are guided on the rear of the machine through partitions (6) in which each hose runs.

7. Dual-purpose machine for air freshening and hygienic cleansing, according to claim 1, **characterised in that** it has a housing (4) for batteries and a housing (5) for the motor.

8. Dual-purpose machine for air freshening and hygienic cleansing, according to claim 1, **characterised in that** it has a led (8) that indicates the level of liquid in the bottles (3.1, 3.2).

9. Dual-purpose machine for air freshening and hygienic cleansing, according to claim 1, **characterised in that** the outer casing (7) has an orifice (7.1) for outlet of the pulverised air freshening liquid and another orifice (7.2) for the indicator led (8).

10. Dual-purpose machine for air freshening and hygienic cleansing, according to claim 1, **characterised In that** all internal elements of the machine are attached to the casing by clips.

## Patentansprüche

1. Zweifach verwendbares Gerät zur Erfrischung und hygienischen Reinigung der Luft, das zwei Flaschen (3.1, 3.2) und einen elektrischen Motor besitzt, **gekennzeichnet durch**
- einen Mechanismus zur Reduzierung der Geschwindigkeit (1), der von dem elektrischen Motor angetrieben wird;
- einen Wechselschalter (1.1), dessen Bewegung von der Drehrichtung des Motors abhängt; und
- zwei Bedienungselemente (2.1, 2.2), welche von dem Wechselschalter (1.1) gepresst werden, was den Ausstoß des Inhalts jeweils einer der beiden Flaschen (3.1, 3.2) verursacht.

2. Zweifach verwendbares Gerät zur Erfrischung und hygienischen Reinigung der Luft nach Anspruch 1, **dadurch gekennzeichnet, dass** der Mechanismus zur Reduzierung der Geschwindigkeit (1) aus einem Antriebsrad (1.2) mit einem Ritzel (1.3) auf dessen Achse besteht, das in ein Zwischenritzel (1.4) eingreift, welches ebenfalls über ein Ritzel (1.5) auf dessen Achse verfügt, das den Wechselschalter (1.1) antreibt.

3. Zweifach verwendbares Gerät zur Erfrischung und hygienischen Reinigung der Luft nach Anspruch 2, **dadurch gekennzeichnet, dass** der Wechselschalter (1.1) die Form eines kreisförmigen Abschnitts mit einem oberen Abschnitt (1.1.1) hat, der gezahnt ist und in das Antriebsritzel (1.5) des Zwischenritzels eingreift, wobei die Enden des unteren Teils des Schalters (1.1) zwei Verlängerungen (1.1.2) besitzen, die als Schalter dienen.

4. Zweifach verwendbares Gerät zur Erfrischung und hygienischen Reinigung der Luft nach Anspruch 1, **dadurch gekennzeichnet, dass** eine der Flaschen (3.1) einen Lufterfrischer und die andere Flasche (3.2) ein hygienisches Reinigungsmittel enthält.

5. Zweifach verwendbares Gerät zur Erfrischung und hygienischen Reinigung der Luft nach Anspruch 4, **dadurch gekennzeichnet, dass** die Flasche (3.2), welche das hygienische Reinigungsmittel enthält, eine Pipette (2.2) mit zwei ausgerichteten vertikalen Auslässen (2.2.1, 2.2.2) besitzt, mit denen ein flexibler Schlauch verbunden ist.

6. Zweifach verwendbares Gerät zur Erfrischung und hygienischen Reinigung der Luft nach Anspruch 5, **dadurch gekennzeichnet, dass** die flexiblen Schläuche auf der Rückseite des Geräts durch Teilungen (6) geführt werden, durch die jeder Schlauch verläuft.

7. Zweifach verwendbares Gerät zur Erfrischung und hygienischen Reinigung der Luft nach Anspruch 1, **dadurch gekennzeichnet, dass** dieses über ein Gehäuse (4) für Batterien und ein Gehäuse (5) für den Motor verfügt.

8. Zweifach verwendbares Gerät zur Erfrischung und hygienischen Reinigung der Luft nach Anspruch 1, **dadurch gekennzeichnet, dass** dieses eine LED (8) besitzt, das den Flüssigkeitsstand in den Flaschen (3.1, 3.2) anzeigt.

9. Zweifach verwendbares Gerät zur Erfrischung und hygienischen Reinigung der Luft nach Anspruch 1, **dadurch gekennzeichnet, dass** das äußere Gehäuse (7) eine Öffnung (7.1) für den Auslass der pulverisierten Flüssigkeit zur Lufterfrischung und eine andere Öffnung (7.2) für die LED-Anzeige (8) besitzt.

10. Zweifach verwendbares Gerät zur Erfrischung und hygienischen Reinigung der Luft nach Anspruch 1, **dadurch gekennzeichnet, dass** alle inneren Elemente des Geräts mittels Clips an dem Gehäuse befestigt sind.

## Revendications

1. Appareil à double fonctionnalité désodorisant et purificateur, doté de deux bouteilles (3.1, 3.2) et d'un moteur électrique **caractérisé par**
Un système de ralentissement (1) actionné par un moteur électrique ;
Un bouton va et vient (1.1), dont la position varie en fonction du sens de rotation du moteur ; et
Deux actionneurs (2.1, 2.2), sur lesquels le bouton va et vient (1.1) exerce une pression provoquant l'expulsion de l'une des deux bouteilles (3.1, 3.2), respectivement.

2. Appareil à double fonctionnalité désodorisant et purificateur, selon la revendication 1, **caractérisé en ce que** le système de ralentissement (1) est constitué d'une roue d'entraînement (1.2) comprenant un pignon (1.3) situé sur l'arbre, qui fait tourner un pignon intermédiaire (1.4) dont l'arbre est également pourvu d'un pignon (1.5) qui entraîne un bouton va et vient (1.1).

3. Appareil à double fonctionnalité désodorisant et purificateur, selon la revendication 2, **caractérisé en ce que** bouton va et vient (1.1), est en forme de secteur circulaire dont le secteur supérieur (1.1.1) est dentelé et actionne le pignon de commande (1.5) du pignon intermédiaire, les extrémités de la partie inférieure du bouton (1.1) possédant deux extensions (1.1.2) et jouant le rôle d'un bouton.

4. Appareil à double fonctionnalité désodorisant et purificateur, selon la revendication 1, **caractérisé en ce que** l'une des bouteilles (3.1) contient un désodorisant et **en ce que** l'autre bouteille (3.2) contient un purificateur.

5. Appareil à double fonctionnalité désodorisant et purificateur, selon la revendication 4, **caractérisé en ce que** la bouteille (3.2) contenant le purificateur possède une pipette (2.2) dotée de deux orifices d'émission verticaux et alignés (2.2.1, 2.2.2) auxquels est relié un tuyau souple.

6. Appareil à double fonctionnalité désodorisant et purificateur, selon la revendication 5, **caractérisé en ce que** les tuyaux souples sont dirigés à l'arrière de l'appareil à l'intérieur de sections (6) à l'intérieur desquelles sont placés chacun des tuyaux.

7. Appareil à double fonctionnalité désodorisant et purificateur, selon la revendication 1, **caractérisé en ce qu'**il comprend un logement (4) pour les piles et un logement (5) pour le moteur.

8. Appareil à double fonctionnalité désodorisant et purificateur, selon la revendication 1, **caractérisé en ce qu'**il comprend un affichage (8) qui indique le niveau de liquide dans les bouteilles (3.1, 3.2).

9. Appareil à double fonctionnalité désodorisant et purificateur, selon la revendication 1, **caractérisé en ce que** le cadre extérieur (7) comprend un orifice (7.1) destiné à l'expulsion du liquide désodorisant en pulvérisation ainsi qu'un autre orifice (7.2) pour l'indicateur d'affichage (8).

10. Appareil à double fonctionnalité désodorisant et purificateur, selon la revendication 1, **caractérisé en ce que** l'ensemble des éléments internes de l'appareil est relié au logement au moyen de clips.
